# EUROPEAN PATENT SPECIFICATION

(11) **EP 4 126 138 B1**
(45) Date of publication and mention of the grant of the patent: **16.10.2024**
(21) Application number: 21714210.8
(22) Date of filing: 29.03.2021
(51) Int. Cl.: A61M 15/00, A61M 11/00

(54) **AEROSOL DELIVERY OF AT LEAST TWO LIQUID COMPOSITIONS**
AEROSOLABGABE VON MINDESTENS ZWEI FLÜSSIGEN ZUSAMMENSETZUNGEN
DISTRIBUTION D'AÉROSOL D'AU MOINS DEUX COMPOSITIONS LIQUIDES

(30) Priority: 31.03.2020 EP 20167140; 31.03.2020 US 202063002836 P
(43) Date of publication of application: 08.02.2023
(73) Proprietor: invoX Belgium NV, 3590 Diepenbeek (BE)
(72) Inventor: RAWERT, Jürgen, 50933 Köln (DE); BARTELS, Frank, 45527 Hattingen (DE); DUDLEY, Steven, Brighton BN1 8NF (GB)
(74) Representative: Synergy IP Group AG
(86) International application number: PCT/EP2021/058109
(87) International publication number: WO 2021/198154

(56) References cited:
- WO-A1-02/04055
- WO-A1-2005/107837
- WO-A1-2018/234525
- WO-A1-2020/030682
- US-A1- 2009 216 183

## Description

### FIELD OF THE INVENTION

The present invention relates to the field of inhalation devices for medically active liquids. In particular, the invention relates to an inhalation device for the generation of an aerosol, the device comprising at least a first reservoir and a second reservoir containing a first liquid composition and a second liquid composition.

### BACKGROUND OF THE INVENTION

Nebulizers or other aerosol generators for liquids are known from the art since a long time ago. Amongst others, such devices are used in medical science and therapy. There, they serve as inhalation devices for the application of active ingredients in the form of aerosols, i.e., small liquid droplets embedded in a gas. Such an inhalation device is known, for example from the document EP 0 627 230 B1. Essential components of this inhalation device are a reservoir in which the liquid that is to be aerosolized is contained; a pumping unit for generation of a pressure being sufficiently high for nebulizing; as well as an atomizing device in the form of a nozzle. A pumping unit is defined as a unit or device component capable of moving or compressing a fluid material and that comprises at least one pumping chamber, and optionally further comprises auxiliary components as well, such as a body, interfaces, and the like. By means of the pumping unit, the liquid is drawn in a discrete amount, i.e., not continuously, from the reservoir, and fed to the nozzle. The pumping unit works without propellant and generates pressure mechanically.

A known embodiment of such an inhalation device is presented in document WO 91/14468 A1. In such a device, the pressure in the pumping chamber which is connected to the housing is generated by movement of a moveable hollow piston. The piston is moveably arranged inside the immobile pumping chamber. The (upstream arranged) inlet of the hollow piston is fluidically connected to the interior of the reservoir (reservoir pipe section). Its (downstream arranged) tip leads into the pumping chamber. Furthermore, a check valve that inhibits a back flow of liquid into the reservoir is arranged inside the tip of the piston.

For filling the piston, the same is directly connected with its upstream end to the reservoir. By pulling out the piston of the pumping chamber, its interior volume is enlarged, such that an increasing relative negative pressure (i.e., partial vacuum) is built up inside the pumping chamber. This pressure propagates through the hollow piston into the reservoir, such that liquid is sucked from the same into the piston. At the same time, said valve opens at its tip, since the pressure inside the reservoir is higher than inside the (yet empty) pumping chamber. The pumping chamber is being filled. At the same time, a spring is loaded, and locked at the motion's end when the moveable piston has reached its lower dead center and the pumping chamber is filled.

The spring can be manually unlocked. The stored energy is then abruptly released. The piston is again pushed in the direction of the pumping chamber and into the same, thus decreasing its interior volume. The aforementioned check valve is now closed, such that a growing pressure builds up inside the pumping chamber, since the liquid is inhibited from flowing back into the reservoir. Eventually, this pressure results in ejection of the liquid from the nozzle which is arranged at the downstream end of the pumping chamber.

In order to face the risk of a reverse flow of already ejected liquid or even outside air, a further check valve, subsequently being called outlet valve, can be arranged at the downstream end of the pumping chamber just before the nozzle, allowing emitted liquid to pass, but blocking incoming gas.

The piston is arranged inside the pressure spring, which is designed as helical spring, thus limiting its outside diameter. Also because of the typically small volume (e.g., 15 µl), the piston is designed with a thin interior (and often also exterior) diameter.

This typically small inner diameter of the moveable piston (e.g., 0.3 to 1.0 mm), together with a small size of the check valve being arranged within, is a drawback of the described construction. The small diameter results in a high flow resistance, such that in particular, media of higher viscosities flow into and through the piston only very slowly. In other words, the described construction is suitable especially for low-viscosity (aqueous) liquids and for emitting low doses thereof. Furthermore, fabrication of a sufficiently tight check valve of small diameter is difficult.

Another disadvantage of the described solution is that only one type of liquid can be emitted at a time, i.e., depending on the content of the reservoir. If another liquid shall be aerosolized, the reservoir must be exchanged, and the nozzle must be cleaned from remnants of the previous liquid before the inhalation device can be used again.

From document EP 1 747 035 B1, an inhalation device is known which is based on the technique described above, but which comprises two separate reservoirs that are connected via two separate pumping mechanisms to two individual ejection nozzles. These nozzles can form two individual sprays consisting of said two liquids, or they can form a single spray that consists of these two liquids. However, the aforementioned drawbacks still apply. Furthermore, the inhalation device contains only one unit (e.g., a spring) that serves as the potential energy source for both reservoirs and pumping mechanisms. As a consequence, the two liquids to be dispensed must have similar properties, e.g., viscosities, as the force that will expel the two liquids will be the same.

Inhalation therapies of more than one drug are also known. In particular, triple combination therapies of a long-acting muscarinic antagonist ("LAMA"), a long-acting beta agonist ("LABA"), and an inhaled corticosteroid ("ICS") have been developed to treat patients with chronic obstructive pulmonary disease ("COPD"). Typically, the LAMA and LABA components are aqueous soluble, whereas the ICS component is soluble in organic solvents and insoluble in aqueous solution. Therefore, patients would need to use two to three different inhalers to dispense the components in liquid form as combining all three components into one solution is not possible without precipitation of at least one of the components. Alternatively, therapies combined in one device have been limited to dry powder or powder suspensions due to the incompatibility of triple solution formulations of LAMA + LABA + ICS.

It is an object of the present invention to provide an improved method for delivering liquid solutions having different properties and/or incompatible ingredients to a patient by inhalation that overcomes one or more of the disadvantages of currently known inhalation therapies. It is a further object to provide an inhalation device that enables such improved delivery method. Further objects of the invention will be clear on the basis of the following description of the invention, examples and claims. WO2018/234525A1 discloses an inhalation device for the generation of an aerosol of at least two liquid compositions with a single potential energy storage unit coupled to an end of both pumping chambers.

### SUMMARY OF THE INVENTION

In a first aspect, the invention relates to an inhalation device for the generation of an aerosol, the device comprising at least a first reservoir and a second reservoir containing a first liquid composition and a second liquid composition, wherein the inhalation device is a pump-actuated inhaler adapted to release upon actuation of a first potential energy storage unit and a second potential energy storage unit a metered dose of the first liquid composition and the second liquid composition from the first reservoir and the second reservoir through a first exit area and a second exit area. In another aspect, the present invention provides for a first reservoir and a second reservoir adapted for use with an inhalation device as defined in the embodiments described herein.

In a further aspect, the invention relates to an inhalation device according to the first aspect of the invention and as defined in the embodiments described herein, for use in the treatment or prevention of a disease or condition, in some embodiments a lung disease or condition. In yet a further aspect, the invention provides an improved method of delivering at least two liquid compositions to a subject according to which an inhalation device as described above is used for the administration of a first liquid composition and a second liquid composition having different properties and/or incompatible ingredients. In yet a further aspect, the invention provides a method of treating a subject which is based on such improved method of delivering at least two liquid compositions.

### DETAILED DESCRIPTION OF THE INVENTION

The objects are solved by the subject-matter of the independent claims. Advantageous embodiments are described in the dependent claims, the subsequent description, as well as the accompanying figures.

Introductorily, some definitions of terms are given which are used throughout the description and claims. The definitions should be used to determine the meaning of the respective expressions unless the context requires a different meaning.

An "inhaler" or "inhaler device" or "inhalation device" is a device which is configured and adapted for the generation of an inhalable mist, vapor, or spray.

The term "about" or the like in connection with an attribute or value includes the exact attribute or precise value, as well as any attribute or value typically considered to fall within the normal or accepted variability associated with the technical field, and methods of measuring or determining said attribute or value.

"Atomization" and "nebulization" in the context of inhalers means the generation of fine, inhalable droplets of a liquid. The typical dimensions of atomized droplets are in the range of several microns.

An "aerosol" is a dispersion of a solid or liquid phase in a gas phase. The dispersed phase, also termed the discontinuous phase, is comprised of multiple solid or liquid particles. The aerosol generated by the inhalation device of the invention is a dispersion of a liquid phase in the form of inhalable liquid droplets in a gas phase which is typically air. The dispersed liquid phase may optionally comprise solid particles dispersed in the liquid.

A "cartridge" is a unit that houses one or more reservoirs that may be removed from the device and replaced when the reservoirs are empty. For example, a cartridge may house one reservoir containing one liquid composition. Alternatively, the cartridge may house at least two reservoirs, each containing a liquid composition, in one unit.

An "exit area" is a location where liquid exits the inhaler device upon actuation of the device. Multiple exit areas may be included in a single nozzle unit. Alternatively, each exit area may be included in its own separate nozzle unit. Each exit area may have one or more channels to eject a liquid composition from the device. For example, an exit area may only comprise one channel or may comprise at least two channels.

A "liquid" is a fluid material capable of altering its shape to that of a container which holds the liquid but retains a nearly constant volume independent of pressure. A liquid may represent a monophasic liquid solution or a dispersion with a continuous liquid phase and a dispersed phase which may or may not be liquid.

A "plurality" means two or more.

"Interior" means inside, but also, oriented towards the inside; "Exterior" means outside, but also, oriented towards the outside.

A "nozzle" is a unit that serves for the atomization/nebulization of liquid. Generally, the term means the unit in its entirety. However, a nozzle can comprise one or multiple sets of individual, identical or different sub-units. A nozzle may have a plurality of ejection channels for emitting the liquid compositions described herein.

The "main axis" of a nozzle is its central axis parallel or collinear to the direction into which the bulk of the emitted aerosol travels after leaving the nozzle.

The "ejection trajectory" is an imaginary and relatively straight line that starts at the end of an ejection channel. It resembles the initial travel path of a liquid emitted from the ejection channel when the inhalation device is operated. It is clear that the nozzle (and the entire inhalation device) must be adapted and configured by means of e.g., a suitable channel geometry and a sufficiently high pressure such that the emitted liquid can be provided in said straight line and with a sharp stream.

Where two or more ejection trajectories intersect, a "collision point" is formed.

The term "metered dose" refers to the amount, for example as defined in terms of volume (e.g., µL, microliter), of liquid composition that is releasable by the inhalation device as a result of a single (one) actuation of the device.

The term "a single dose" in reference to a composition refers to the complete amount of the composition administered to a subject in a dosing event, and which is pharmacologically active which is administered as part of a dosing regimen. As understood herein, a single dose may be administered to a subject, via a single (one) actuation of the inhalation device; alternatively, it may also be administered over a plurality of actuations of the inhalation device, for example 2, or 3, or 4 actuations of the device, and in accordance with the prescribed usage, whereby the metered doses released per individual actuation combine to provide the required single dose.

The term "comprising" and related terms "comprise" or "comprises" would be understood as meaning that features additional to the features prefaced by the term may be present. Conversely, the term "consists" and related terms would be understood as meaning that no other features, other than those prefaced by the term are present, and if present, only in trace or residual amounts such as to confer no technical advantage or relevance in respect of the object of the invention.

Further definitions are provided in the subsequent description.

In a first aspect, the invention relates to an inhalation device for the generation of an aerosol of at least two liquid compositions, the device comprising at least a first and second reservoir containing at least a first and a second liquid composition, wherein the inhalation device is a pump-actuated inhaler adapted to release upon actuation a metered dose of the first liquid composition from the first reservoir and a metered dose of the second liquid composition from the second reservoir, wherein the first and second reservoir for storing the first and second liquid composition are fluidically connected to at least a first and second pumping chamber for generation of a first pressure inside said first pumping chamber and a second pressure inside said second pumping chamber, a first and second riser pipe, each of which can be received with a reservoir-facing, interior end in said first and second pumping chamber, wherein the interior volume of the first and second pumping chamber is changeable by means of relative motion of the first and second pumping chamber to the first and second riser pipe, at least a first exit area and a second exit area adapted to separately eject the first and second liquid composition from the inhalation device, a first potential energy storage unit coupled with one end of the first pumping chamber, and a second potential energy storage unit coupled with one end of the second pumping chamber.

The inhalation device according to the invention serves for the generation of an aerosol of at least two liquid compositions (e.g., a first liquid composition and a second liquid composition), in particular, of such aerosols which can be inhaled by a subject, e.g., a warm-blooded animal or human, especially a human subject, in need thereof. Preferably, the inhalation device is suited to be hand-held and/or portable and may also be used by the individual subject themselves, following prescribed instructions. In some embodiments, the first reservoir and the second reservoir are incorporated into a single cartridge unit or the first reservoir is incorporated into a first cartridge unit and the second reservoir is incorporated into a second cartridge unit. Such cartridge unit(s) may be removed from the device and replaced when the reservoirs are empty after use.

The inhalation device is pump-actuated inhaler which as understood herein is an inhalation device comprising at least two mechanically driven pump devices or units that are each capable of displacing or compressing a liquid and/or fluid, such as a first liquid composition and a second liquid composition each having different properties and different active ingredients, additives, and/or excipients according to the present invention. In one embodiment of the invention, the pump-actuated inhaler is a piston-pump actuated inhaler having at least two piston-pump systems and at least two potential energy storage units each coupled to the piston-pump systems.

The pump-actuated inhaler may comprise at least two pump devices or units, each coupled to a separate potential energy storage unit, which generate a desired pressure for releasing at least two compositions having different properties and active ingredients, additives, and/or excipients, such as in the present aspect, through a first exit area and a second exit area, generating at least two aerosol streams. In some preferred embodiments, the at least two aerosol streams are separated upon injection to prevent undesirable chemical interactions or precipitation of the active ingredients, additives, and/or excipients. By means of the at least two pumping units, each of the first liquid composition and second liquid composition is drawn in a discrete amount, i.e., not continuously, from the first reservoir and the second reservoir, and fed to the first exit area and the second exit area. The at least two pumping units work without propellant and generate pressure mechanically. The at least two pump units or devices each preferably comprise of a pumping chamber, and a means for the storage of potential energy, each device being coupled to the pumping chamber and being lockable in a loaded position, wherein upon unlocking, the stored energy is transformable into a motion of the pumping chamber. The inhaler device comprises at least two potential energy storage units comprising, such as, for example, a spring, a gas, or a magnetic force. Because each pump unit is coupled to a separate potential energy storage unit, each pump unit may be configured to expel each liquid composition with different forces or different volumes, to compensate for any differences in properties (e.g., viscosities) of the individual liquid compositions to be dispensed.

In specific embodiments, the at least two, or more specifically, the two potential energy storage units may each be in the form of a spring, wherein each of the at least two, or more specifically, two springs, may have the same or a different spring rate (k), preferably a different spring rate, typically selected within the range of from about 50 N/m (Newton/meter) to about 5.000 (five thousand) N/m, such as from about 100 N/m to about 2.000 N/m, or from about 150 N/m to about 1.500 N/m.

In further specific embodiments, the two springs may produce the same or a different force typically ranging from about 5 N to about 200 N (Newton), or from about 10 N to about 100 N, or from about 20 N to about 80 N, or from about 30 N to about 70 N or from about 37 N to about 62 N. In further specific embodiments, however, the two springs may produce different forces with a first spring having a force selected within the range of from about 80 N to about 120 N, and a second spring having a force selected within the range of from about 40 N to about 80 N.

The two potential energy storage units, especially when in the form of spring such as a spiral spring may be made of a suitable elastic material, such as metal, for example, steel, copper, beryllium-copper alloys, nickel alloys, especially nickel-chromium alloys, such as Inconel^{®}, or from another material, for example, a polymeric or ceramic material, such as, for example, zirconia oxide or aluminum oxide or rubber, for example rubber springs. In specific embodiments, the two potential energy storage units may be preferably made from the same material or may be made from two different materials.

In some embodiments, the at least two exit areas of the inhalation device each eject the first liquid composition and the second liquid composition along respective ejection trajectories. In some embodiments, the first exit area and the second exit area are incorporated into a single nozzle, or the first exit area is incorporated into a first nozzle and the second exit area is incorporated into a second nozzle. In some embodiments, the first exit area comprises at least two channels to eject the first liquid composition from the device and the second exit area comprises at least two channels to eject the second liquid composition from the device. In some particular embodiments, the first liquid composition is ejected from at least two ejection channels, each having its own ejection trajectory, and the second liquid composition is ejected from at least two ejection channels, each having its own ejection trajectory and independent of the ejection channels of the first liquid composition, for a total of at least four exit areas for one inhaler device. In some particular embodiments, for each liquid composition, the at least two ejection channels may be oriented at angles such that at least two of said ejection trajectories intersect with one another at a collision point, such that a collision-type (or impingement-based) aerosol formation is achieved.

In some preferred embodiments, the exit areas and/or nozzles are adapted to separately eject the first liquid composition and the second liquid composition to avoid any interference, undesired chemical interactions, or precipitation of the potentially incompatible components (e.g., active ingredients, additives, and/or excipients) of the liquid compositions. In some embodiments, the first liquid composition is ejected from one ejection channel having its own ejection trajectory, i.e., a direction along which the respectively emitted liquid stream leaves its channel, and the second liquid composition is ejected from another ejection channel having its own ejection trajectory. Alternatively, the first liquid composition is ejected from at least two ejection channels each having its own ejection trajectory, and the second liquid composition is ejected from at least two ejection channels each having its own ejection trajectory. In preferred embodiments, the exit areas and/or nozzles are configured such that no interference of the aerosol streams of the at least two liquid compositions occur upon ejection from the device.

In specific embodiments, the inhalation device according to the present invention may be to about -mist-inhaler (SMI) wherein the term "soft-mist-inhaler" as used herein, refers to a preferably non-electrified mobile inhalation device for liquid formulations with low velocity nebulization properties. In further specific embodiments, such inhalation device or, more specifically, such soft-mist-inhaler comprises at least one impingement-type nozzle as described above for the nebulization/aerosolization of the medically active liquids to be administered.

Accordingly, in further specific embodiments, the inhalation device according to the present invention is a soft-mist inhaler and comprises at least two or, more specifically two impingement-type nozzles as described above, each impingement-type nozzle having two ejection channels with two corresponding ejection trajectories, wherein the two ejection trajectories of one impingement-type nozzle have one collision point in which the two ejection trajectories intersect. In further specific embodiments, each of the at least two liquid compositions or, more specifically, of the first and the second liquid compositions is dispensed or ejected by a separate impingement-type nozzle as described above.

Preferably, the inhalation device according to the invention and the embodiments described herein releases a metered dose each of the first and second liquid compositions upon actuation, each metered dose having the same or different volume and is at least about 1 µL (microliter). In further embodiments, each of the metered doses of the first and second liquid composition released upon actuation of the inhalation device may have the same or different volume and is at least about 1 µL, 2 µL, 5 µL, 10 µL, or 15 µL, or at least about 20 µL, 25 µL, 30 µL, or 50 µL. In other embodiments, each of the metered doses of the first and second liquid compositions released upon actuation may have the same or different volume and is about 1 µL to about 50 µL, or about 5 to about 30 µL, or about 10 to about 20 µL. In some embodiments, each of the metered doses of the first and second liquid compositions released upon actuation has a volume of about 1 µL. In other embodiments, each of the metered doses of the first and second liquid compositions released upon actuation has a volume of about 10 µL. In yet other embodiments, each of the metered doses of the first and second liquid compositions released upon actuation has a volume of about 15 µL.

In another embodiment of the invention, a single dose of the first liquid composition and the second liquid composition may be released by a one (single) actuation, or a plurality of actuations of the inhalation device.

Preferably each of the single doses of the first liquid composition and the second liquid composition may have the same or different mass and comprises an amount of at least about 0.1 µg (microgram), such as from about 0.1 µg to about 1.000 µg, or from about 1 µg to about 250 µg, or from about 1 µg to about 50 µg of a first active ingredient and at least about 0.1 µg, such as from about 0.1 µg to about 1.000 µg, or from about 1 µg to about 250 µg or from about 1 µg to about 50 µg of a second active ingredient. In other embodiments, the first liquid composition comprises a first active ingredient and the second liquid composition comprises a second active ingredient that may have the same or different mass and comprises an amount of at least 1 µg, 2 µg, 2.5 µg, 5 µg, 10 µg, 20 µg, 50 µg, or 100 µg. In yet further embodiments, the single dose of the first liquid composition comprises an amount of a first active ingredient and the second liquid composition comprises an amount of a second active ingredient and optionally a third active ingredient that may have the same or different mass in the range of about 1 to 10 µg, about 2 to 30 µg, or about 2.5 to 25 µg.

In further embodiments, the first and/or the second liquid composition, as the case may be, may comprise more than one active ingredient, such as a mixture of two or more different active ingredients in the amounts or concentrations, as described above.

In a further embodiment, the inhalation device can be held comfortably with one hand. The inhalation device comprises at least two reservoirs for separately storing the first and second liquid compositions, and at least two pumping units each comprising a pumping chamber for generation of a pressure inside said pumping chamber, wherein the first pumping chamber is fluidically connected with the first reservoir, optionally by means of a first reservoir pipe (or reservoir pipe section), via a first check valve, which blocks in the direction of the first reservoir. Similarly, the second pumping chamber is fluidically connected with the second reservoir, optionally by means of a second reservoir pipe (or reservoir pipe section), via a second check valve, which blocks in the direction of the second reservoir. Thus, the first and second check valves allow a liquid flow from the first and second reservoirs into the first and second pumping chambers and block a flow in the opposite direction.

In some embodiments, the inhalation device further comprises a first riser pipe having at least one reservoir-facing, interior end which can be received in the first pumping chamber, and a first exit area, which is connected liquid-tight directly or indirectly to the exterior end of the first riser pipe. Similarly, the inhalation device further comprises a second riser pipe having at least one reservoir-facing, interior end which can be received in the second pumping chamber, and a second exit area, which is connected liquid-tight directly or indirectly to the exterior end of the second riser pipe.

In some embodiments, the interior volume of the at least two pumping chambers are changeable by means of relative motion of the pumping chamber to the riser pipes in that each riser pipe increases the volume by being pushed into and decreases the volume by being pulled out of its respective pumping chamber. The term "interior volume" describes the volume which extends from the reservoir-facing inlet of each pumping chamber to the place where the interior end of the respective riser pipe is located.

According to the invention, each riser pipe is immobile and firmly, directly or indirectly, and/or permanently or detachably, attached to the device, while each pumping chamber is moveable relative to the device. In other words, each riser pipe maintains its position relative to the device, and each pumping chamber can alter its position relative to the device, and in particular, along a longitudinal axis of the same, such as to perform a piston-in-cylinder-type movement of the immobile riser pipe in the moveable pumping chamber. Such an immobile riser pipe is described in detail in WO2018/197730.

In another embodiment, the immobility of each riser pipe is primarily related to the exit area, rather than to the device. Thus, exit areas and riser pipes form - in terms of movability - one unit. However, if the exit areas itself are immobile with respect to the device, this is also true for the riser pipes, thus arriving at the described embodiment.

An advantage of these features is that the passages between pumping chambers and reservoirs can be designed with less restrictions compared to the known art. It is, for example possible to design a significantly larger check valve, which is easier to manufacture, since it does not have to be contained within the hollow piston known from the art. As a result, the size of the respective check valve is mainly only restricted by the interior size of the device or, if such a construction is desired, the inner size of a potential energy storage unit that surrounds the pumping units. The (approximate) identity of the diameter of valve, riser pipe and reservoir pipe, as known from the art, becomes obsolete. Furthermore, since no movable piston needs to be connected to each of the respective reservoirs, the component which enters the reservoirs and the moveable component (i.e., the pumping chambers) can be designed independent of each other, allowing to better suit the individual functions. In this respect, the invention provides for higher design flexibility because the at least two moveable pumping chambers, due to their robust structure and dimensions, provide better opportunities for designing a mechanically stable connection with the reservoirs than does the respective moveable riser pipe which is typically less robust. Also, the connection between pumping chambers and reservoirs can be designed with a larger diameter, such that higher flow velocities and fluid viscosities become feasible. Further, a mechanical support for the reservoirs can be integrated into the component that comprises the pumping chambers. Additionally, the vent for pressure equilibration of the reservoirs can be moved away from the reservoir body itself to, e.g., a connector which forms an interface between reservoirs and pumping chambers, facilitating the construction and avoiding the necessity to provide an essentially "open" reservoir body.

According to one embodiment, each of the at least two check valves is adapted to open only when the pressure difference between the upstream and the downstream side of the valve, i.e., the reservoir and the pumping chamber side, is above a predefined threshold value, and remains closed as long as the pressure difference is below the threshold value. "Pressure difference" means that, irrespective of the concrete pressure values, only the relative pressure difference between the two sides is relevant for determining whether the check valve blocks or opens.

Upon activation of each of the at least two pumping units, by building up a high pumping chamber pressure, the pressure difference (due to a high pressure in the pumping chamber, and a significantly lower pressure in the reservoir, resulting in a large pressure difference) becomes high enough and exceeds the threshold value of the pressure difference, so that each of the at least two check valves finally opens and allows the pressure chamber being filled with liquid from the reservoir.

According to a further embodiment, an inhalation device comprises at least two outlet valves, one inside each of the at least two riser pipes for avoiding a return flow of liquid or air into the exterior end of the same.

According to another embodiment, the inhalation device comprises at least two outlet valves between each of the at least two riser pipes and at least two exit areas for avoiding a return flow of liquid or air towards the exterior end of each of the at least two riser pipes.

Optionally, each of the at least two outlet valves can be of a type that blocks below (and opens above) a threshold pressure difference as described above.

In some embodiments, the first exit area and the second exit are incorporated into a single nozzle, or the first exit area is incorporated into a first nozzle and the second exit area is incorporated into a second nozzle in order to separate the two exit streams of the first and second liquid compositions. In particular embodiments, the nozzle(s) is constructed as a stack of relatively flat plates. Such plates can preferably be fabricated by material subtracting technologies such as etching or the like. Wafers of different materials such as silicon, glass, metal, ceramics, or plastics can form the semi-finished product. The channels leading to each exit area are brought into one of the two flat sides of the substrate, or even on both sides. Then, by stacking several of such plates, a nozzle stack providing a plurality of ejection channel pairs can be fabricated. In some embodiments, each exit area comprises at least two channels to eject the liquid compositions from the device.

In other embodiments, the nozzle(s) is constructed from a three-dimensional rotation symmetric basic shape. Such a basic shape can be a cone, a cylinder, or a pyramid. Typically, the rotation or symmetry axis of the base shape coincides with the main axis of the finished nozzle.

According to a further embodiment, each of the at least two reservoirs are firmly attached to the pumping chamber and thus moveable inside the device. This means that in each ejection cycle, the first reservoir moves together with the first pumping chamber from the initial position, in which the first pumping chamber has its maximum interior volume, to the end position, in which the same is minimal, and eventually back to the initial position. Similarly, the second reservoir moves together with the second pumping chamber from the initial position, in which the second pumping chamber has its maximum interior volume, to the end position, in which the same is minimal, and eventually back to the initial position. As used herein, the expression "firmly attached" includes both permanent and non-permanent (i.e., releasable) forms of attachment. One of the advantages of this construction is that it provides the smallest possible dead volume between reservoirs and pumping chambers.

According to another embodiment, each of the at least two reservoirs is connected to the at least one pumping chamber by means of a flexible element such as e.g., a hose, and firmly attached to the device. Thus, according to this embodiment, each of the first and second reservoirs does not move along with the first and second pumping chambers, but is firmly (but, however, typically detachably) attached to the device. One advantage of this construction is that the energy which is abruptly released upon unlocking the means for the storage of potential energy acts solely onto the first and second pumping chambers for accelerating the same, but not also onto the first and second reservoirs which typically - and in particular at the beginning of its usage - can have a relatively large mass. A higher acceleration of the first and second pumping chambers, and thus, a higher pressure, is the result.

In another aspect, the present invention relates to a first reservoir containing a first liquid composition comprising a long acting beta agonist, and a second reservoir containing a second liquid composition comprising an inhaled corticosteroid, wherein each of the first and second reservoirs is adapted for use with an inhalation device according to any one of the claims 1 to 17. The first and second reservoirs are adapted to be housed and integrated with the other features and components of the inhalation device and may be standardized where a plurality of reservoirs (e.g., two or greater than two) may be contained and integrated in the inhalation device. Said first reservoir is connectable to the first pumping chamber, and said second reservoir is connectable to the second pumping chamber. In one embodiment, each of the reservoirs (2A, 2B) according to the invention is adapted to be firmly attachable to each of the pumping chambers (3A, 3B) and is thus moveable inside the device. In an alternative embodiment, each of the reservoirs (2A, 2B) is adapted to connect to each of the pumping chamber (3A, 3B) by means of a flexible element, and firmly attached to the device.

The amount of the first and second liquid compositions which may be accommodated, i.e., stored in the first and second reservoirs is an amount whereby on actuation of the device, an amount of at least one metered dose of the first and second liquid compositions is released. In some embodiments, each of the first and second reservoir is a multi-dose reservoir, meaning that it contains a plurality of single doses, which may be administered via a plurality of actuations of the device. Alternatively expressed, each of the first and second reservoirs preferably contains an amount of the first and second liquid compositions adapted for multiple, or a plurality of actuations of the inhalation device.

In one embodiment, the inhalation device and/or the at least two reservoirs adapted for the inhalation device contains an amount of at the least two liquid compositions suitable for 1 to about 120, or 1 to about 90, or 1 to about 60, or 1 to about 30, or 1 to about 20, or from about 10 to about 90 or to about 60 actuations of the inhalation device, or an amount of at least two liquid compositions suitable for the release of 1 to 120, or 1 to 90, or 1 to 60, or 1 to 30, or 1 to 20, or from about 10 to about 60 or to about 80 metered doses of aerosolized liquid compositions.

In a further aspect of the present invention, the inhaler device includes a first liquid composition in a first reservoir and a second liquid composition in a second reservoir. The first and second liquid compositions are formulated as compositions that are suitable, and adapted for inhalative use, in other words compositions that may be atomized for inhalation and that are physiologically acceptable for inhalation by a subject.

The first and second liquid compositions contained within the inhalation device, and, more specifically, within the first and second reservoirs may be in the form of a dispersion, for example a suspension with a liquid continuous phase, and a solid dispersed phase. Preferably, however, the first and second liquid compositions are in the form of a solution, where active ingredients and other materials are dissolved and solubilized in a liquid carrier solution.

In some embodiments, the first and the second liquid compositions comprise the same or different solvent compositions as liquid carriers. In specific embodiments, however, the first and the second liquid compositions preferably comprise different solvent compositions as liquid carriers. For example, the first liquid composition and second liquid composition may both comprise an aqueous solution. In other embodiments, the first liquid composition may comprise an aqueous solution, and the second liquid composition may comprise an organic solution or a mixture of aqueous and organic solutions. In some embodiments, the organic solution comprises an alcoholic solution, such as an ethanolic solution.

In further specific embodiments, the liquid carrier or solvent comprises water and/or ethanol, preferably ethanol. In further specific embodiments, such liquid carrier or solvent comprises or preferably consists of ethanol or a mixture of ethanol and water, wherein the ethanol may be comprised in an amount of at least about 50 wt.-%, or at least about 60 wt.-% or at least about 70 wt.-% or even more and water in a corresponding amount of up to about 50 wt.-%, or up to about 40 wt.-% or up to about 30 wt.-% or less. In specific embodiments, the liquid vehicle or solvent comprises or consists of ethanol in an amount of about 60 to about 80 wt.-%, such as about 70 wt.-%, and water in an amount of about 40 to about 20 wt.-%, such as about 30 wt.-%.

In some embodiments, the first liquid composition comprises a long-acting beta agonist (LABA), preferably in a pharmaceutically effective amount. Non-limiting, exemplary long-acting beta agonists include albuterol, arformoterol, bambuterol, bitolterol, broxaterol, carbuterol, clenbuterol, fenoterol, formoterol, hexoprenaline, ibuterol, indacaterol, indacterol, isoetharine, isoprenaline levosalbutamol, mabuterol meluadrine, metaproterenol, olodaterol, orciprenaline, pirbuterol, procaterol, reproterol, rimiterol, ritodrine, salmeterol, salmefamol, soterenot, sulphonterol, tiaramde, terbutaline, and terbuterol. In some embodiments, the long-acting beta agonist is olodaterol.

In other embodiments, the first liquid composition comprises a long-acting muscarinic antagonist (LAMA), preferably in a pharmaceutically effective amount. Non-limiting, exemplary long-acting muscarinic antagonists include aclidinium bromide, glycopyrronium bromide, revefenacin, tiotropium, such as tiotropium bromide, umeclidinium bromide, oxitropium bromide, flutropium bromide, ipratropium bromide, trospium chloride, and tolterodine. In some embodiments, the long-acting muscarinic antagonist is tiotropium bromide.

In some embodiments, the first liquid composition comprises a mixture of a long-acting beta agonist and a long-acting muscarinic antagonist. In some embodiments, the mixture of the long-acting beta agonist and long-acting muscarinic antagonist comprises olodaterol and tiotropium bromide.

In some embodiments, the second liquid composition comprises an inhaled corticosteroid (ICS). Non-limiting, exemplary inhaled corticosteroids include prednisolone, prednisone, butixocort propionate, flunisolide, beclomethasone, triamcinolone, budesonide, fluticasone, mometasone (furoate), ciclesonide, rofleponide, dexamethasone, etiprednol-dichloroacetat, deflazacort, etiprednol, loteprednol, RPR-106541, NS-126, and ST-26. In some embodiments, the inhaled corticosteroid is ciclesonide.

In some embodiments, the first liquid composition comprises a long-acting beta agonist and the second liquid composition comprises an inhaled corticosteroid. In specific embodiments, the long-acting beta agonist is olodaterol and the inhaled corticosteroid is ciclesonide.

In some embodiments, the first liquid composition comprises a long-acting beta agonist and the second liquid composition comprises an inhaled corticosteroid and a metered dose of the first liquid composition comprising the long-acting beta agonist has a volume of at least 1 µL and a metered dose of the second liquid composition comprising the inhaled corticosteroid has a volume of at least 1 µL. In some embodiments, the metered dose of the first liquid composition comprises an amount of at least 1 µg of the long-acting beta agonist, and wherein the metered dose of the second liquid composition comprises an amount of at least 1 µg of the inhaled corticosteroid.

In some embodiments, the long-acting beta agonist is dissolved in the first liquid composition, and the inhaled corticosteroid is dissolved in the second liquid composition.

In some embodiments, the first liquid composition comprises a mixture of a long-acting beta agonist and a long-acting muscarinic antagonist and the second liquid composition comprises an inhaled corticosteroid. In specific embodiments, the mixture of the long-acting beta agonist and long-acting muscarinic antagonist comprises olodaterol and tiotropium bromide and the inhaled corticosteroid is ciclesonide. In particular embodiments, a metered dose of the first liquid composition comprising the long-acting beta agonist and long-acting muscarinic antagonist has a volume of at least 1 µL and a metered dose of the second liquid composition comprising the inhaled corticosteroid has a volume of at least 1 µL. In some embodiments, the metered dose of the first liquid composition comprises an amount of at least 1 µg of the long-acting beta agonist and an amount of at least 1 µg of the long-acting muscarinic antagonist, and wherein the metered dose of the second liquid composition comprises an amount of at least 1 µg of the inhaled corticosteroid.

In some embodiments, the long-acting beta agonist is dissolved in the first liquid composition, and the inhaled corticosteroid is dissolved in the second liquid composition and long-acting muscarinic antagonist is dissolved in the first liquid composition.

In further embodiments, the first and second liquid compositions may comprise, optionally, one or more physiologically acceptable additives and/or excipients, which are suitable for inhalative use. Excipients which may be featured in the composition include, but are not limited to, one or more buffering agents to regulate or control pH of the solution, salts, taste-masking agents, surfactants, lipids, antioxidants, preservatives, such as benzalkonium chloride (BAC), parabens such as methylparaben, ethylparaben, propylparaben, sodium benzoate, sorbic acid and salts thereof, and co-solvents, which may be used to enhance or improve solubility, for example ethanol, or a glycol. In some embodiments, the liquid compositions are also essentially free of a propellant such as a hydrofluoroalkane (HFA) propellant.

A further aspect of the present invention relates to the use of the inhalation device in therapy and prophylactic treatment of a disease or condition. In specific embodiments, the disease or condition is a lung or respiratory disease or condition. In particular, the inhalation device according to the invention is used for the treatment or prevention of a lung disease or condition. As understood herein, a lung disease or condition may affect one or more anatomical aspect and/or function of a subject's lung(s) and associated respiratory airways.

Treatment refers to the administration of the first and second liquid compositions for the therapy of the disease or condition, for example leading to ameliorating, decreasing, or relieving of at least one symptom of the disease or condition, or stopping or slowing the progression of at least one symptom of the disease or condition, such as, for example, preserving lung function. The prevention of a disease or condition may be understood to be prophylactic treatment and refers to the administration of the first and second liquid compositions to a subject that may not have developed the disease or condition but is at risk, or susceptible to the disease or condition.

In both the treatment or prevention of the disease or condition, the first and second liquid compositions are administered by means of the inhalation device and embodiments described herein at therapeutically effective amounts, such as in the amounts described above.

In a particularly preferred embodiment, the disease or condition is a lung disease such as, for example, asthma or chronic obstructive pulmonary disease ("COPD").

Described herein is the use of an inhalation device or reservoirs containing the first and second liquid compositions, as described in any one of the above embodiments, in the manufacture or preparation of a medicament or medical device for the treatment of a subject in need thereof in relation to any disease or condition is also provided for in the context of the present invention. In particular embodiments, the disease or condition is a lung disease or condition, for example, asthma or chronic obstructive pulmonary disease ("COPD"). In other particular embodiments, the first liquid composition comprises a long-acting beta agonist, such as olodaterol and the second liquid composition comprises an inhaled corticosteroid, such as ciclesonide. In yet other particular embodiments, the first liquid composition further comprises a long-acting muscarinic antagonist, such as tiotropium bromide.

Further described herein is a method of treating or preventing a disease or condition in a subject in need thereof, the method comprising a step of administering a metered dose of a first liquid composition and a second liquid composition using an inhalation device as described in any one or combination of the above embodiments. In particular embodiments, the disease or condition is a lung disease or condition, for example, asthma or chronic obstructive pulmonary disease ("COPD"). In other particular embodiments, the first liquid composition comprises a long-acting beta agonist, such as olodaterol and the second liquid composition comprises an inhaled corticosteroid, such as ciclesonide. In yet other particular embodiments, the first liquid composition further comprises a long-acting muscarinic antagonist, such as tiotropium bromide.

Preferably the inhalation device used in the methods described herein is a handheld, i.e., portable device, whereby the administration of the first and second liquid compositions and actuation of the device is carried out by the human subject or patient themselves directly and in accordance with prescribed instructions which may also accompany the device.

Also described herein is a method for delivering liquid compositions to a subject in need thereof, the method comprising the step of providing an inhalation device as described above to said subject. The subject is preferably a human patient, in particular a human patient suffering from a disease or condition, preferably a lung disease, such as asthma or COPD. The patient may further be provided with instructions to use the device, to actuate it and to inhale the aerosol emitted from it.

In yet a further aspect, the invention provides a method of treating a subject suffering from a disease or condition, preferably a lung disease, such as asthma or COPD, the method comprising a step of administering a first and second liquid composition to said subject using a device as described above. Again, the patient may be provided with instruction to use the device, to actuate it, and to inhale the aerosol emitted from it.

Also described is the provision of the combination of a first and a second liquid composition for use in a method of treating a subject suffering from a disease or condition, preferably a lung disease, such as asthma or COPD, wherein the first and the second liquid composition is administered to the subject using an inhalation device according to the first aspect of the invention.

### DESCRIPTION OF THE DRAWINGS

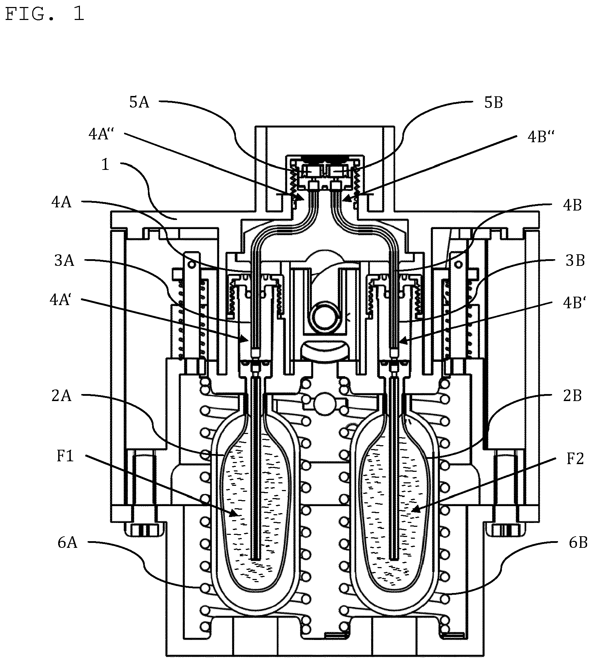

- **Figure** 1: shows the main components of an inhalation device useful for carrying out the invention.

In **Figure 1****,** the main components of an exemplary inhalation device useful for carrying out the invention are depicted schematically and not-to-scale, at the situation prior to first use. The depicted device represents a non-limiting embodiment of the inhalation devices of the present invention disclosed herein.

The inhalation device comprises a housing 1, which is preferably shaped and dimensioned such that it can be held with one hand and can be operated by one finger, e.g., the thumb (not shown). The device further comprises two reservoirs 2A, 2B for the respective storage of a medically active liquid F1, F2. The depicted reservoirs 2A, 2B are designed to be collapsible; that means that during proceeding emptying, the elastic or at least limp walls buckle, so that the relative negative pressure (i.e., partial vacuum) which is necessary for extraction of a certain amount of liquid F1, F2 is not, or almost not, increased. A similar effect can be achieved via alternative embodiments, in which a rigid container has a moveable bottom by means of which the interior volume of the respective reservoir can also be successively be reduced (not shown).

Further, the inhalation device comprises a pumping unit with two pumping chambers 3A, 3B for generation of the desired pressures which are necessary for emitting liquid F1, F2 and nebulizing the same. The pumping unit can also comprise additional components that are not depicted (push button, locking device, etc.).

At least two potential energy storage units 6A, 6B (e.g., springs) are provided, each of which is coupled with one (upwards directed) end to the pumping chambers 3A, 3B and which is supported at housing 1 (lower part of the figure).

The inhalation device further comprises at least two riser pipes 4A, 4B with at least one respective reservoir-facing, interior end 4A', 4B' which can be received in said pumping chambers 3A, 3B. In other words, riser pipes 4A, 4B can at least partially be pushed into pumping chambers 3A, 3B, resulting in a decrease of the interior volumes of pumping chambers 3A, 3B. The term "interior volume" describes that volume which extends from the reservoir-facing inlet of the pumping chamber 3A, 3B to the place where the interior end 4A', 4B' of the riser pipe 4A, 4B is located.

Preferably, in the section which serves for the reception of the riser pipes, pumping chamber 3A, 3B has section with a circular inner cross section that corresponds to the (then also) circular outside cross section of the according riser pipe section. Of course, other cross section shapes are possible as well.

Further, the inhalation device comprises two exit areas 5A, 5B, each of which is connected liquid-tight to the respective exterior ends 4A", 4B" of riser pipes 4A, 4B. Exit areas 5A, 5B are suitable for nebulizing / atomizing liquid in a single stream or at least two streams by using the principle of two colliding liquid jets. Exit areas 5A, 5B which are depicted as an example comprises two separate nozzle units, but the exit areas 5A, 5B may also be included in one nozzle unit (not depicted). Each of the two exit areas 5A, 5B is connected to an individual pumping chamber 3A, 3B and thus, liquid reservoirs 2A, 2B. Each liquid F1, F2 has its own pumping chamber 3A, 3B in order to avoid undesired mixing.

Riser pipes 4A, 4B are designed to be immobile and firmly attached to the device. Riser pipes 4A, 4B are also firmly attached to exit areas 5A, 5B, which in turn are attached to the device as well. On the contrary, pumping chambers 3A, 3B are designed to be moveable with respect to the device and exit areas 5A, 5B. The benefits of this design have already been explained; reference is made to the respective sections above.

### LIST OF REFERENCES

- 1: housing
- 2A,2B: first and second reservoirs
- 3A,3B: first and second pumping chambers
- 4A,4B: first and second riser pipes
- 4A',4B': interior end
- 4A",4B": exterior end
- 5A,5B: first exit area and second exit area
- 6A,6B: potential energy storage units
- F1,F2: first and second liquid compositions

## Claims

1. An inhalation device for the generation of an aerosol of at least two liquid compositions, the device comprising at least a first and second reservoir (2A, 2B) containing at least a first and a second liquid composition (F1, F2), wherein the inhalation device is a pump-actuated inhaler adapted to release upon actuation a metered dose of the first liquid composition (F1) from the first reservoir (2A) and a metered dose of the second liquid composition (F2) from the second reservoir (2B), wherein the first and second reservoir (2A, 2B) for storing the first and second liquid composition (F1, F2) are fluidically connected to at least a first and second pumping chamber (3A, 3B) for generation of a first pressure inside said first pumping chamber (3A) and a second pressure inside said second pumping chamber (3B), a first and second riser pipe (4A, 4B), each of which can be received with a reservoir-facing, interior end (4A', 4B') in said first and second pumping chamber (3A, 3B), wherein the interior volume of the first and second pumping chamber (3A, 3B) is changeable by means of relative motion of the first and second pumping chamber (3A, 3B) to the first and second riser pipe (4A, 4B), at least a first exit area and a second exit area (5A, 5B) adapted to separately eject the first and second liquid composition (F1, F2) from the inhalation device, a first potential energy storage unit (6A) coupled with one end of the first pumping chamber (3A), and a second potential energy storage unit (6B) coupled with one end of the second pumping chamber (3B), wherein each of the first and second riser pipe (4A, 4B) is immobile and firmly attached to the device and/or to the first and second exit area (5A, 5B), and each of the first and second pumping chamber (3A, 3B) is movable relative to the device and/or to the first and second exit area (5A, 5B).

2. The inhalation device according to claim 1, wherein the first and second pumping chamber (3A, 3B) coupled to a separate potential energy storage unit (6A, 6B) generates a desired pressure for releasing the at least two liquid compositions.

3. The inhalation device according to claim 1 or 2, wherein the first potential energy storage unit (6A) and the second potential energy storage unit (6B) each produce the same force, or wherein the first potential energy storage unit (6A) and the second potential energy storage unit (6B) each produce a different force.

4. The inhalation device according to claim 3, wherein the force is about 5 newtons (N) to about 200 newtons (N).

5. The inhalation device according to any one of claims 1 to 4, wherein the first potential energy storage unit (6A) and the second potential energy storage unit (6B) each comprise a spring, specifically, wherein each spring has the same or different spring rate (k) from about 50 N/m to about 5.000 (five thousand) N/m.

6. The inhalation device according to any one of claims 1 to 5, wherein the metered dose of the first liquid composition (F1) has the same volume than the metered dose of the second liquid composition (F2), orwherein the metered dose of the first liquid composition (F1) has a different volume than the metered dose of the second liquid composition (F2).

7. The inhalation device according to any one of claims 1 to 6, wherein the first liquid composition comprises a long-acting beta agonist and the second liquid composition comprises an inhaled corticosteroid, specifically, wherein the first liquid composition comprises a mixture of a long-acting beta agonist and a long-acting muscarinic antagonist and the second liquid composition comprises an inhaled corticosteroid.

8. The inhalation device according to claim 7, wherein the long-acting beta agonist is selected from the group consisting of albuterol, arformoterol, bambuterol, bitolterol, broxaterol, carbuterol, clenbuterol, fenoterol, formoterol, hexoprenaline, ibuterol, indacaterol, indacterol, isoetharine, isoprenaline levosalbutamol, mabuterol meluadrine, metaproterenol, olodaterol, orciprenaline, pirbuterol, procaterol, reproterol, rimiterol, ritodrine, salmeterol, salmefamol, soterenot, sulphonterol, tiaramde, terbutaline, and terbuterol.

9. The inhalation device according to claim 7 or 8, wherein long-acting muscarinic antagonist is selected from the group consisting of aclidinium bromide, glycopyrronium bromide, revefenacin, tiotropium bromide, umeclidinium bromide, oxitropium bromide, flutropium bromide, ipratropium bromide, trospium chloride, and tolterodine.

10. The inhalation device according to any one of claims 7 to 9, wherein the inhaled corticosteroid is selected from the group consisting of prednisolone, prednisone, butixocort propionate, flunisolide, beclomethasone, triamcinolone, budesonide, fluticasone, mometasone, ciclesonide, rofleponide, dexamethasone, etiprednol-dichloroacetat, deflazacort, etiprednol, loteprednol, RPR-106541, NS-126, and ST-26.

11. The inhalation device according to any one of claims 7 to 10, wherein the long-acting muscarinic antagonist is tiotropium bromide, the long-acting beta agonist is olodaterol, and the inhaled corticosteroid is ciclesonide.

12. The inhalation device according to any one of the preceding claims, wherein the first liquid composition (F1) comprises an aqueous solution and the second liquid composition (F2) comprises an organic solution or a mixture of aqueous and organic solutions, specifically, wherein the organic solution comprises an alcoholic solution.

13. The inhalation device according to any one of the preceding claims, wherein the first exit area (5A) and the second exit area (5B) are incorporated into a single nozzle, orwherein the first exit area (5A) is incorporated into a first nozzle and the second exit area (5B) is incorporated into a second nozzle.

14. The inhalation device according to any one of the preceding claims, wherein the first exit area (5A) comprises at least two channels to eject the first liquid composition (F1) from the device and the second exit area (5B) comprises at least two channels to eject the second liquid composition (F2) from the device.

15. The inhalation device according to any one of the preceding claims, wherein the first reservoir and the second reservoir are incorporated into a single cartridge unit, or wherein the first reservoir is incorporated into a first cartridge unit and the second reservoir is incorporated into a second cartridge unit.

16. The inhalation device according to any one of the preceding claims, wherein the inhalation device is a soft-mist-inhaler, preferably
wherein the soft-mist inhaler comprises at least two impingement-type nozzles, each impingement-type nozzle having two ejection channels with two corresponding ejection trajectories, wherein the two ejection trajectories of one impingement-type nozzle have one collision point in which the two ejection trajectories intersect.

17. The inhalation device according to any one of the preceding claims for use in the treatment or prevention of disease or condition, specifically for use in the treatment or prevention of a lung disease or condition such as asthma or chronic obstructive pulmonary disease ("COPD").

18. A first reservoir containing a first liquid composition comprising a long-acting beta agonist and a second reservoir containing a second liquid composition comprising an inhaled corticosteroid, wherein the first and second reservoirs are adapted for use with an inhalation device according to any one of the preceding claims, preferably wherein the first liquid composition further comprises a long-acting muscarinic antagonist.

## Patentansprüche

1. Inhalationsvorrichtung zur Erzeugung eines Aerosols aus mindestens zwei flüssigen Zusammensetzungen, wobei die Vorrichtung mindestens ein erstes und ein zweites Reservoir (2A, 2B) umfasst, die mindestens eine erste und eine zweite flüssige Zusammensetzung (F1, F2) enthalten, wobei es sich bei der Inhalationsvorrichtung um einen pumpenbetätigten Inhalator handelt, der dazu ausgelegt ist, bei Betätigung eine abgemessene Dosis der ersten flüssigen Zusammensetzung (F1) aus dem ersten Reservoir (2A) und eine abgemessene Dosis der zweiten flüssigen Zusammensetzung (F2) aus dem zweiten Reservoir (2B) abzugeben, wobei das erste und das zweite Reservoir (2A, 2B) zum Speichern der ersten und der zweiten flüssigen Zusammensetzung (F1, F2) fluidisch mit mindestens einer ersten und einer zweiten Pumpkammer (3A, 3B) zur Erzeugung eines ersten Drucks innerhalb der ersten Pumpkammer (3A) und eines zweiten Drucks innerhalb der zweiten Pumpkammer (3B) verbunden sind, sowie ein erstes und ein zweites Steigrohr (4A, 4B), von denen jedes mit einem dem Reservoir zugewandten inneren Ende (4A', 4B') in der ersten und zweiten Pumpkammer (3A, 3B) aufgenommen werden kann, wobei das Innenvolumen der ersten und zweiten Pumpkammer (3A, 3B) mittels einer Relativbewegung der ersten und zweiten Pumpkammer (3A, 3B) zu dem ersten und zweiten Steigrohr (4A, 4B) veränderbar ist, sowie mindestens einen ersten Austrittsbereich und einen zweiten Austrittsbereich (5A, 5B), die dazu ausgelegt sind, die erste und zweite flüssige Zusammensetzung (F1, F2) getrennt aus der Inhalationsvorrichtung auszustoßen, sowie eine erste potenzielle Energiespeichereinheit (6A), die mit einem Ende der ersten Pumpkammer (3A) gekoppelt ist, und eine zweite potenzielle Energiespeichereinheit (6B), die mit einem Ende der zweiten Pumpkammer (3B) gekoppelt ist, wobei sowohl das erste als auch das zweite Steigrohr (4A, 4B) unbeweglich und fest mit der Vorrichtung und/oder dem ersten und zweiten Austrittsbereich (5A, 5B) verbunden ist, und sowohl die erste als auch die zweite Pumpkammer (3A, 3B) relativ zu der Vorrichtung und/oder dem ersten und zweiten Austrittsbereich (5A, 5B) beweglich ist

2. Inhalationsvorrichtung nach Anspruch 1, wobei die erste und zweite Pumpkammer (3A, 3B), die mit einer separaten potenziellen Energiespeichereinheit (6A, 6B) gekoppelt sind, einen gewünschten Druck zur Freisetzung der mindestens zwei flüssigen Zusammensetzungen erzeugen.

3. Inhalationsvorrichtung nach Anspruch 1 oder 2, wobei die erste potenzielle Energiespeichereinheit (6A) und die zweite potenzielle Energiespeichereinheit (6B) jeweils die gleiche Leistung erbringen, oder wobei die erste potenzielle Energiespeichereinheit (6A) und die zweite potenzielle Energiespeichereinheit (6B) jeweils eine unterschiedliche Leistung erbringen.

4. Inhalationsvorrichtung nach Anspruch 3, wobei die Leistung etwa 5 Newton (N) bis etwa 200 Newton (N) beträgt

5. Inhalationsvorrichtung nach einem der Ansprüche 1 bis 4, wobei die erste potenzielle Energiespeichereinheit (6A) und die zweite potenzielle Energiespeichereinheit (6B) jeweils eine Feder umfassen, wobei insbesondere jede Feder die gleiche oder eine unterschiedliche Federkonstante (k) von etwa 50 N/m bis etwa 5.000 (fünftausend) N/m aufweist.

6. Inhalationsvorrichtung nach einem der Ansprüche 1 bis 5, wobei die abgemessene Dosis der ersten flüssigen Zusammensetzung (F1) das gleiche Volumen aufweist wie die abgemessene Dosis der zweiten flüssigen Zusammensetzung (F2) oder wobei die abgemessene Dosis der ersten flüssigen Zusammensetzung (F1) ein unterschiedliches Volumen aufweist als die abgemessene Dosis der zweiten flüssigen Zusammensetzung (F2).

7. Inhalationsvorrichtung nach einem der Ansprüche 1 bis 6, wobei die erste flüssige Zusammensetzung einen langfristig wirkenden Beta-Agonisten und die zweite flüssige Zusammensetzung ein inhalatives Kortikosteroid umfasst, wobei insbesondere die erste flüssige Zusammensetzung ein Gemisch aus einem langfristig wirkenden Beta-Agonisten und einem langfristig wirkenden Muscarin-Antagonisten umfasst und die zweite flüssige Zusammensetzung ein inhalatives Kortikosteroid umfasst

8. Inhalationsvorrichtung nach Anspruch 7, wobei der langfristig wirkende Beta-Agonist ausgewählt ist aus der Gruppe bestehend aus Albuterol, Arformoterol, Bambuterol, Bitolterol, Broxaterol, Carbuterol, Clenbuterol, Fenoterol, Formoterol, Hexoprenalin, Ibuterol, Indacaterol, Indacterol, Isoetharin, Isoprenalin, Levosalbutamol, Mabuterol, Meluadrin, Metaproterenol, Olodaterol, Orciprenalin, Pirbuterol, Procaterol, Reproterol, Rimiterol, Ritodrin, Salmeterol, Salmefamol, Soterenot, Sulfonterol, Tiaramde, Terbutalin und Terbuterol.

9. Inhalationsvorrichtung nach Anspruch 7 oder 8, wobei der langfristig wirkende Muscarin-Antagonist ausgewählt ist aus der Gruppe bestehend aus Aclidiniumbromid, Glycopyrroniumbromid, Revefenacin, Tiotropiumbromid, Umeclidiniumbromid, Oxitropiumbromid, Flutropiumbromid, Ipratropiumbromid, Trospiumchlorid und Tolterodin.

10. Inhalationsvorrichtung nach einem der Ansprüche 7 bis 9, wobei das inhalative Kortikosteroid ausgewählt ist aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Etiprednol-Dichloracetat, Deflazacort, Etiprednol, Loteprednol, RPR-106541, NS-126 und ST-26.

11. Inhalationsvorrichtung nach einem der Ansprüche 7 bis 10, wobei es sich bei dem langfristig wirkenden Muscarin-Antagonisten um Tiotropiumbromid, bei dem langfristig wirkenden Beta-Agonisten um Olodaterol und bei dem inhalativen Kortikosteroid um Ciclesonid handelt

12. Inhalationsvorrichtung nach einem der vorstehenden Ansprüche, wobei die erste flüssige Zusammensetzung (F1) eine wässrige Lösung und die zweite flüssige Zusammensetzung (F2) eine organische Lösung oder ein Gemisch aus wässrigen und organischen Lösungen umfasst, wobei insbesondere die organische Lösung eine alkoholische Lösung umfasst.

13. Inhalationsvorrichtung nach einem der vorstehenden Ansprüche, wobei der erste Austrittsbereich (5A) und der zweite Austrittsbereich (5B) in eine einzige Düse integriert sind, oder wobei der erste Austrittsbereich (5A) in eine erste Düse und der zweite Austrittsbereich (5B) in eine zweite Düse integriert ist

14. Inhalationsvorrichtung nach einem der vorstehenden Ansprüche, wobei der erste Austrittsbereich (5A) mindestens zwei Kanäle zum Ausstoßen der ersten flüssigen Zusammensetzung (F1) aus der Vorrichtung umfasst und der zweite Austrittsbereich (5B) mindestens zwei Kanäle zum Ausstoßen der zweiten flüssigen Zusammensetzung (F2) aus der Vorrichtung umfasst

15. Inhalationsvorrichtung nach einem der vorstehenden Ansprüche, wobei das erste Reservoir und das zweite Reservoir in einer einzigen Kartuscheneinheit enthalten sind, oder wobei das erste Reservoir in einer ersten Kartuscheneinheit und das zweite Reservoir in einer zweiten Kartuscheneinheit enthalten ist

16. Inhalationsvorrichtung nach einem der vorstehenden Ansprüche, wobei es sich bei der Inhalationsvorrichtung um einen Vernebelungsinhalator handelt, vorzugsweise wobei der Vernebelungsinhalator mindestens zwei Zerstäuberdüsen umfasst, wobei jede Zerstäuberdüse zwei Ausstoßkanäle mit zwei entsprechenden Ausstoßbahnen aufweist, wobei die beiden Ausstoßbahnen einer Zerstäuberdüse einen Kollisionspunkt aufweisen, in dem sich die beiden Ausstoßbahnen schneiden.

17. Inhalationsvorrichtung nach einem der vorstehenden Ansprüche zur Verwendung bei der Behandlung oder Vorbeugung einer Krankheit oder eines Leidens, insbesondere zur Verwendung bei der Behandlung oder Vorbeugung einer Lungenkrankheit oder eines Leidens wie Asthma oder chronisch obstruktiver Lungenerkrankung ("COPD").

18. Erstes Reservoir, das eine erste flüssige Zusammensetzung enthält, die einen langfristig wirkenden Beta-Agonisten umfasst, und zweites Reservoir, das eine zweite flüssige Zusammensetzung enthält, die ein inhalatives Kortikosteroid umfasst, wobei das erste und das zweite Reservoir zur Verwendung mit einer Inhalationsvorrichtung nach einem der vorstehenden Ansprüche ausgelegt sind, wobei die erste flüssige Zusammensetzung vorzugsweise ferner einen langfristig wirkenden Muskarin-Antagonisten umfasst.

## Revendications

1. Dispositif d'inhalation destiné à générer un aérosol d'au moins deux compositions liquides, le dispositif comprenant au moins un premier réservoir et un deuxième réservoir (2A, 2B) qui contient au moins une première composition liquide et une deuxième composition liquide (F1, F2), le dispositif d'inhalation étant un inhalateur actionné par pompe ui est adapté pour libérer lors de l'actionnement une dose mesurée de la première composition liquide (F1) à partir du premier réservoir (2A) et une dose mesurée de la deuxième composition liquide (F2) à partir du deuxième réservoir (2B), les premier et deuxième réservoirs (2A, 2B) destinés à stocker les première et deuxième compositions liquides (F1, F2) étant reliés fluidiquement à au moins une première chambre de pompage et une deuxième chambre de pompage (3A, 3B) afin de générer une première pression à l'intérieur de ladite première chambre de pompage (3A) et une deuxième pression à l'intérieur de ladite deuxième chambre de pompage (3B), des premier et deuxième conduits montants (4A, 4B), qui peuvent chacun être reçus avec une extrémité intérieure dirigée vers le réservoir (4A', 4B') dans lesdites première et deuxième chambres de pompage (3A, 3B), le volume intérieur des première et deuxième chambres de pompage (3A, 3B) pouvant être modifié par un mouvement relatif des première et deuxième chambres de pompage (3A, 3B) par rapport aux premier et deuxième conduits montants (4A, 4B), au moins une première zone de sortie et une deuxième zone de sortie (5A, 5B) étannt adaptées pour éjecter séparément les première et deuxième compositions liquides (F1, F2) du dispositif d'inhalation, une première unité de stockage d'énergie potentielle (6A) étant accouplée à une extrémité de la première chambre de pompage (3A), et une deuxième unité de stockage d'énergie potentielle (6B) étant accouplée à une extrémité de la deuxième chambre de pompage (3B), chacun des premier et deuxième conduits montants (4A, 4B) étant immobile et solidement fixé au dispositif et/ou aux première et deuxième zones de sortie (5A, 5B), et chacune des première et deuxième chambres de pompage (3A, 3B) étant mobile par rapport au dispositif et/ou aux première et deuxième zones de sortie (5A, 5B).

2. Dispositif d'inhalation selon la revendication 1, les première et deuxième chambres de pompage (3A, 3B) accouplées à une unité de stockage d'énergie potentielle séparée (6A, 6B) générant une pression souhaitée pour libérer les au moins deux compositions liquides.

3. Dispositif d'inhalation selon la revendication 1 ou 2, la première unité de stockage d'énergie potentielle (6A) et la deuxième unité de stockage d'énergie potentielle (6B) produisant chacune la même force, ou la première unité de stockage d'énergie potentielle (6A) et la deuxième unité de stockage d'énergie potentielle (6B) produisant des forces différentes.

4. Dispositif d'inhalation selon la revendication 3, la force étant d'environ 5 newtons (N) à environ 200 newtons (N).

5. Dispositif d'inhalation selon l'une quelconque des revendications 1 à 4, la première unité de stockage d'énergie potentielle (6A) et la deuxième unité de stockage d'énergie potentielle (6B) comprenant chacune un ressort, en particulier lesdits ressorts des raideurs (k) identiques ou différentes d'environ 50 N/m à environ 5000 (cinq mille) N/m.

6. Dispositif d'inhalation selon l'une quelconque des revendications 1 à 5, la dose mesurée de la première composition liquide (F1) ayant le même volume que la dose mesurée de la deuxième composition liquide (F2), ou la dose mesurée de la première composition liquide (F1) ayant un volume différent de celui de la dose mesurée de la deuxième composition liquide (F2).

7. Dispositif d'inhalation selon l'une quelconque des revendications 1 à 6, la prentmière composition liquide compren un agoniste bêta à action prolongée et la deuxième composition liquide comprenant un corticostéroïde inhalé, en particulier la première composition liquide comprenant un mélange d'un agoniste bêta à action prolongée et d'un antagoniste muscarinique à action prolongée et la deuxième composition liquide comprenant un corticostéroïde inhalé.

8. Dispositif d'inhalation selon la revendication 7, l'agoniste bêta à action prolongée étant choisi dans le groupe comprenant l'albutérol, l'arformotérol, le bambutérol, le bitoltérol, le broxatérol, le carbutérol, le clenbutérol, le fénotérol, le formotérol, l'hexoprénaline, l'ibutérol, l'indacatérol, l'indacterol, l'isoétharine, l'isoprénaline. lévosalbutamol, le mabutérol méluadrine, le métaprotérénol, l'olodatérol, l'orciprénaline, le pirbutérol, le procatérol, le reprotérol, le rimitérol, la ritodrine, le salmétérol, le salméfamol, le sotérénot, le sulfontérol, le tiaramde, la terbutaline et le terbutérol.

9. Dispositif d'inhalation selon la revendication 7, l'antagoniste muscarinique à action prolongée étant choisi dans le groupe comprenant le bromure d'aclidinium, le bromure de glycopyrronium, la révéfénacine, le bromure de tiotropium, le bromure d'uméclidinium, le bromure d'oxitropium, le bromure de flutropium, le bromure d'ipratropium, le chlorure de trospium et la toltérodine.

10. Dispositif d'inhalation selon l'une quelconque des revendications 7 à 9, le corticostéroïde inhalé étant choisi dans le groupe comprenant la prednisolone, la prednisone, le propionate de butixocort, le flunisolide, la béclométhasone, la triamcinolone, le budésonide, la fluticasone, la mométasone, la ciclésonide, le rofléponide, la dexaméthasone, l'étiprednoldichloroacétate, le déflazacort, l'étiprednol, le lotéprednol, le RPR-106541, le NS-126 et le ST-26.

11. Dispositif d'inhalation selon l'une quelconque des revendications 7 à 10, l'antagoniste muscarinique à action prolongée étant le bromure de tiotropium, l'agoniste bêta à action prolongée étant l'olodatérol et le corticostéroïde inhalé étant le ciclésonide.

12. Dispositif d'inhalation selon l'une quelconque des revendications précédentes, la première composition liquide (F1) comprenant une solution aqueuse et la deuxième composition liquide (F2) comprenant une solution organique ou un mélange de solutions aqueuse et organique, en particulier, la solution organique comprenant une solution alcoolique.

13. Dispositif d'inhalation selon l'une quelconque des revendications précédentes, la première zone de sortie (5A) et la deuxième zone de sortie (5B) étant incorporées dans une seule buse, ou la première zone de sortie (5A) étant incorporée dans une première buse et la deuxième zone de sortie (5B) étant incorporée dans une deuxième buse.

14. Dispositif d'inhalation selon l'une quelconque des revendications précédentes, la première zone de sortie (5A) comprenant au moins deux canaux afin d'éjecter la première composition liquide (F1) du dispositif et la deuxième zone de sortie (5B) comprenant au moins deux canaux afin d'éjecter la deuxième composition liquide (F2) du dispositif.

15. Dispositif d'inhalation selon l'une quelconque des revendications précédentes, le premier réservoir et le deuxième réservoir étant incorporés dans une seule unité formant cartouche, ou le premier réservoir étant incorporé dans une première unité formant cartouche et le deuxième réservoir étant incorporé dans une deuxième unité formant cartouche.

16. Dispositif d'inhalation selon l'une quelconque des revendications précédentes, le dispositif d'inhalation étant un inhalateur à brouillard doux, de préférence l'inhalateur à brouillard doux comprenant au moins deux buses de type à impact, chaque buse de type à impact comportant deux canaux d'éjection ayant deux trajectoires d'éjection correspondantes, les deux trajectoires d'éjection d'une buse de type à impact ayant un point de collision dans lequel les deux trajectoires d'éjection se croisent.

17. Dispositif d'inhalation selon l'une quelconque des revendications précédentes destiné à être utilisé dans le traitement ou la prévention d'une maladie ou d'un état, spécifiquement destiné à être utilisé dans le traitement ou la prévention d'une maladie ou d'un état pulmonaire tel que l'asthme ou la bronchopneumopathie chronique obstructive (« BPCO »).

18. Premier réservoir contenant une première composition liquide comprenant un agoniste bêta à action prolongée et un deuxième réservoir contenant une deuxième composition liquide comprenant un corticostéroïde inhalé, les premier et deuxième réservoirs étant adaptés pour être utilisés avec un dispositif d'inhalation selon l'une quelconque des revendications précédentes, de préférence la première composition liquide comprenant en outre un antagoniste muscarinique à action prolongée.
